# FASCICULE DE BREVET EUROPEEN

(11) **EP 3 268 345 B1**
(45) Date de publication et mention de la délivrance du brevet: **24.04.2019**
(21) Numéro de dépôt: 16712960.0
(22) Date de dépôt: 04.03.2016
(51) Int. Cl.: C07C 51/44, C07C 57/04

(54) **PROCEDE AMELIORE DE PRODUCTION D'ACIDE (METH)ACRYLIQUE**
VERBESSERTES VERFAHREN ZUR HERSTELLUNG VON (METH)ACRYLSÄURE
IMPROVED PROCESS FOR PRODUCING (METH)ACRYLIC ACID

(30) Priorité: 12.03.2015 FR 1552049
(43) Date de publication de la demande: 17.01.2018
(73) Titulaire: Arkema France, 92700 Colombes (FR)
(72) Inventeur: JAIN, Sandeep, 75003 Paris (FR); LACROIX, Christian, 57600 Forbach (FR); FAUCONET, Michel, 57730 Valmont (FR)
(74) Mandataire: Bonnel, Claudine
(86) Numéro de dépôt international: PCT/FR2016/050501
(87) Numéro de publication internationale: WO 2016/142608

(56) Documents cités:
- US-B2- 7 288 169

## Description

### DOMAINE TECHNIQUE

La présente invention concerne la production d'acide (méth)acrylique.

Elle a plus particulièrement pour objet un procédé de récupération/purification d'acide (méth)acrylique qui n'utilise pas de solvant azéotropique et basé sur la mise en oeuvre de deux colonnes de purification d'un mélange réactionnel comprenant de l'acide (méth)acrylique. Le procédé selon l'invention inclut un système de condensation à pompe à vide sèche, permettant de réduire la quantité de rejets aqueux ultimes.

L'invention a trait également à une installation adaptée pour la mise en oeuvre de ce procédé.

### ARRIERE-PLAN TECHNIQUE ET PROBLEME TECHNIQUE

Les industriels ont développé depuis des décennies des procédés de synthèse de l'acide acrylique.

Le procédé, qui aujourd'hui est le plus largement exploité industriellement, met en oeuvre une réaction d'oxydation catalytique du propylène en présence d'oxygène.

Cette réaction est conduite généralement en phase gazeuse, et le plus souvent en deux étapes : la première étape réalise l'oxydation sensiblement quantitative du propylène en un mélange riche en acroléine, puis, lors de la deuxième étape réalise l'oxydation sélective de l'acroléine en acide acrylique.

Les conditions de réaction de ces deux étapes, réalisées dans deux réacteurs en série ou dans un seul réacteur contenant les 2 étapes de réaction en série, sont différentes et nécessitent des catalyseurs adaptés à la réaction ; il n'est cependant pas nécessaire d'isoler l'acroléine intermédiaire au cours de ce procédé en deux étapes.

Le mélange gazeux issu de la deuxième étape est constitué, en dehors de l'acide acrylique :
- des impuretés issues de la première étape de réaction qui n'ont pas réagi ;
- de composés légers incondensables dans les conditions de température et de pression habituellement mises en oeuvre, non transformés dans la première étape ou formés dans la seconde étape : azote, oxygène non converti, monoxyde et dioxyde de carbone formés en faible quantité par oxydation ultime ou tournant en rond, par recyclage, dans le procédé ;
- de composés légers condensables non transformés dans la première étape ou formés dans la seconde étape : l'eau, l'acroléine non converti, des aldéhydes légers comme le formaldéhyde et l'acétaldéhyde, l'acide formique, l'acide acétique ou l'acide propionique ;
- de composés lourds : furfuraldéhyde, benzaldéhyde, acide et anhydride maléique, acide benzoïque, acide 2-buténoïque, phénol, protoanémonine.

La complexité du mélange gazeux obtenu dans ce procédé nécessite de procéder à un ensemble d'opérations pour récupérer l'acide acrylique contenu dans cet effluent gazeux et le transformer en un grade d'acide acrylique compatible avec son utilisation finale, par exemple la production de polymères d'acide acrylique, ou la production de polymères d'esters acryliques.

La première étape de cette phase de récupération/purification consiste en une extraction de l'acide acrylique par absorption à contre-courant dans un solvant, généralement de l'eau amenée par une source extérieure et/ou provenant du procédé. Les quantités d'eau et de mélange réactionnel gazeux sont telles que la teneur massique en acide acrylique dans la solution aqueuse brute produite est généralement de l'ordre de 40 à 80%.

Il se pose néanmoins un problème économique très important, principalement en raison de l'énergie coûteuse nécessaire à l'élimination de l'eau utilisée comme solvant d'absorption de l'acide acrylique, dans la mesure où l'élimination efficace de l'eau sans perte exagérée d'acide (méth)acrylique est compliquée par l'existence d'interactions (liaisons hydrogène) entre les 2 composés.

Ainsi, cette opération de séparation est généralement réalisée à l'échelle industrielle par distillation avec un tiers solvant azéotropique, ce qui contribue à augmenter le nombre de colonnes de distillation et leurs coûts énergétiques associés. D'autre part, la multiplication des colonnes de distillation entraine un coût additionnel lié à la consommation supplémentaire d'inhibiteurs de polymérisation qui doivent être introduits sur chacune des dites colonnes, afin de réaliser la purification du produit recherché et l'élimination des sous-produits en évitant les problèmes d'encrassement des appareils par polymérisation du monomère.

Une alternative à ce processus qui utilise l'eau comme solvant d'absorption de l'acide acrylique est d'utiliser un solvant lourd hydrophobe pour extraire l'acide acrylique, mais un tel processus ne simplifie pas le procédé de purification de l'acide acrylique.

Récemment, pour pallier ces différents inconvénients, de nouvelles technologies « sans solvant » de récupération/purification d'acide acrylique sont apparues, impliquant un nombre réduit d'étapes de purification et supprimant l'introduction de solvant organique externe.

Dans le procédé de production d'acide acrylique décrit dans le brevet US 7,151,194, le mélange réactionnel gazeux est envoyé dans une colonne d'absorption et mis en contact avec une solution aqueuse d'absorption, pour obtenir une solution aqueuse d'acide acrylique, qui est ensuite distillée en l'absence de solvant azéotropique. Un flux d'acide acrylique brut est obtenu en pied ou en soutirage latéral de la colonne de distillation, qui est envoyé ensuite sur une unité de purification par cristallisation. Un inconvénient de ce procédé est que l'introduction d'eau extérieure comme solvant d'absorption rend difficile l'élimination de l'eau en tête de colonne d'absorption sans perte importante d'acide acrylique, et la récupération d'une qualité d'acide acrylique brut à faible concentration d'eau en soutirage latéral, lorsque ce procédé est mis en jeu dans une configuration à 2 colonnes.

Le brevet EP 2 066 613 décrit un procédé de récupération d'acide acrylique sans utiliser d'eau extérieure, ni de solvant azéotropique et ne mettant en oeuvre que deux colonnes de purification du mélange réactionnel gazeux refroidi : a) une colonne de déshydratation, b) et une colonne de finition (ou colonne de purification) alimentée par une partie du flux de pied de la colonne de déshydratation.

La colonne de déshydratation fonctionne généralement à la pression atmosphérique ou légèrement supérieure.

Dans la colonne de déshydratation, le flux gazeux distillé en tête est condensé et renvoyé en partie à la colonne de déshydratation sous forme de reflux pour absorber l'acide acrylique.

La colonne de finition fonctionne généralement à une pression inférieure à la pression atmosphérique, permettant d'opérer à des températures relativement faibles, pour éviter ainsi la polymérisation des produits insaturés présents, et minimiser la formation de sous-produits lourds.

Dans la colonne de finition, le distillat de tête comprenant de l'eau et des sous-produits légers est condensé puis recyclé en pied de la première colonne, et un flux comprenant de l'acide acrylique enrichi en sous-produits lourds est éliminé en pied pour être utilisé éventuellement pour la production d'esters acryliques.

Un flux d'acide acrylique purifié correspondant à un grade technique est récupéré par soutirage latéral sous forme de liquide ou de vapeur. L'acide acrylique technique obtenu est généralement de pureté supérieure à 98,5% massique et contient moins de 0,5% massique d'eau.

Dans ce procédé, une partie des flux (du pied de la colonne de déshydratation ou de tête de la colonne de finition) est renvoyée avantageusement aux dispositifs de chauffage/rebouilleur de la colonne de déshydratation et/ou utilisée pour refroidir le mélange réactionnel gazeux, ce qui permet d'optimiser les besoins énergétiques du procédé. Malgré les avantages que procure le procédé décrit dans le document EP 2 066 613, il subsiste encore des inconvénients liés à sa mise en oeuvre.

En particulier, en tête de la colonne de finition qui fonctionne sous vide, le condenseur libère des vapeurs résiduelles comprenant des impuretés organiques qu'il faut éliminer, par exemple par incinération, ce qui est néfaste pour l'environnement.

Les systèmes générateurs de vide disponibles pour réduire la pression de fonctionnement des colonnes de distillation sont nombreux (voir par exemple Techniques de l'Ingénieur, Pompes à vide, B4030, 10/11/1983). On distingue généralement des pompes dites « volumétriques » qui génèrent la dépression en utilisant des joints liquides (huiles, produits organiques ou eau), comme par exemple les pompes à palettes et pompes à anneau liquide, et des pompes dites « à entrainement », dans lesquelles c'est l'écoulement d'un fluide qui crée la dépression (pompes à éjecteurs, à jet de vapeur,...). Les systèmes les plus communément utilisés mettent en jeu des éjecteurs à jet d'eau ou de vapeur d'eau ou des pompes à anneau liquide, principalement à anneau d'eau.

Ces systèmes, ne sont pas adaptés à la réduction de la pression de fonctionnement de la colonne de finition d'un procédé de purification d'acide (méth)acrylique sans solvant, tel que celui décrit dans le document EP 2 066 613.

De tels systèmes, par exemple décrits dans les documents US 6,677,482 ou US 7,288,169, utilisent de la vapeur d'eau et génèrent en quantité importante des effluents aqueux contenant de l'acide acrylique et des impuretés organiques, qui ne peuvent pas être économiquement recyclées dans la boucle de purification formée par la colonne de déshydratation et la colonne de finition. En effet, il a été constaté que le recyclage d'une trop grande quantité d'eau au niveau de la colonne de déshydratation entraine des pertes conséquentes en acide acrylique en tête de cette colonne, à moins d'utiliser une colonne surdimensionnée mais entrainant un investissement coûteux. Ces effluents aqueux nécessitent donc d'être envoyés dans une station de traitement d'eau ou directement dans un oxydeur thermique, occasionnant ainsi d'une part une perte de produit noble et d'autre part un rejet nocif pour l'environnement. Les évents issus de ces systèmes sous vide, riches en composés incondensables, incinérés ou envoyés directement dans l'atmosphère, libèrent dans le premier cas des produits d'oxydation et dans le second cas des composés organiques polluants pour l'environnement.

Il subsiste donc un besoin d'éliminer et/ou réduire la génération de rejets aqueux dans un procédé de récupération/purification sans solvant permettant le fonctionnement sous vide de la colonne de finition.

Les inventeurs ont maintenant découvert que l'utilisation d'une pompe à vide sèche liée au fonctionnement de la colonne de finition dans un procédé de récupération/purification d'acide acrylique sans solvant permet de répondre à ce besoin, avec les avantages économiques et environnementaux en résultant.

Il est par ailleurs apparu aux inventeurs que cette invention pouvait s'appliquer à l'acide acrylique produit à partir d'autres sources que le propylène, à l'acide méthacrylique, ainsi qu'à ces acides dérivés de matières premières renouvelables, qui sont susceptibles de poser les mêmes problèmes de purification.

### RESUME DE L'INVENTION

La présente invention concerne en premier lieu un procédé de récupération d'acide (méth)acrylique sans utiliser de solvant azéotropique, à partir d'un mélange réactionnel gazeux comprenant de l'acide (méth)acrylique obtenu par oxydation en phase gazeuse d'un précurseur de l'acide (méth)acrylique, comprenant au moins les étapes suivantes :
i) on soumet le mélange réactionnel gazeux à une déshydratation sans utiliser de solvant azéotropique dans une première colonne dite colonne de déshydratation, conduisant à un flux de tête dont une partie au moins est condensée et renvoyée à la colonne de déshydratation sous forme de reflux, et à un flux de pied ;
ii) on soumet au moins en partie le flux de pied de colonne de déshydratation à une distillation à une pression inférieure à la pression atmosphérique dans une seconde colonne dite colonne de finition, conduisant à un flux de tête, et à un flux de pied contenant des composés lourds ;
iii) on récupère un flux d'acide (méth)acrylique par soutirage latéral de la colonne de finition, et/ou en pied de la colonne de finition ;
ledit procédé étant caractérisé en ce que le flux de tête de la colonne de finition est au moins en partie soumis à un système de condensation à pompe à vide sèche, formant un condensat qui est renvoyé dans la colonne de déshydratation, et un effluent gazeux ultime.

Dans la présente invention, le terme « (méth)acrylique » signifie « acrylique » ou « méthacrylique ».
Le terme « solvant azéotropique » désigne tout solvant organique présentant la propriété de former un mélange azéotropique avec l'eau.
Le terme « léger » qualifiant les composés sous-produits désigne les composés dont le point d'ébullition est inférieur à celui de l'acide (méth)acrylique, et par analogie, le terme « lourd » désigne les composés dont le point d'ébullition est supérieur à celui de l'acide (méth)acrylique.

Le procédé selon l'invention peut comprendre en outre d'autres étapes visant à poursuivre la purification du flux d'acide (méth)acrylique récupéré à l'étape iii).

Selon certains modes de réalisation particuliers, l'invention présente également une ou, de préférence, plusieurs des caractéristiques avantageuses énumérées ci-dessous :
- le système de condensation à pompe à vide sèche comprend au moins un condenseur et une pompe à vide sèche (pouvant être dénommée aussi pompe à vide sèche primaire) ;
- le système de condensation à pompe à vide sèche peut comprendre de façon optionnelle, en plus de la pompe à vide sèche primaire, un séparateur de liquide, un ou plusieurs pare-flamme, un ou plusieurs filtres, des systèmes d'étanchéité et d'isolement, une pompe ou une combinaison de pompes sèches d'appoint, comme par exemple des pompes de type « Roots » (pompes volumétriques utilisant deux rotors synchronisés tournant en sens inverse) ;
- au moins une partie du flux d'acide (méth)acrylique soutiré latéralement est soumis à un système de condensation à pompe à vide sèche, identique ou différent de celui utilisé pour le flux de tête de la colonne de finition.

Selon un mode réalisation de l'invention, le précurseur de l'acide (méth)acrylique est l'acroléine.

Selon un mode réalisation de l'invention, l'acroléine est obtenue par oxydation de propylène ou par oxydéshydrogénation de propane.

Selon un mode réalisation de l'invention, le précurseur de l'acide (méth)acrylique est la méthacroléine.

Selon un mode réalisation de l'invention, la méthacroléine est obtenue par oxydation d'isobutylène et/ou de tert-butanol.

Selon un mode réalisation de l'invention, la méthacroléine est obtenue à partir d'oxydéshydrogénation de butane et/ou isobutane.

Selon un mode réalisation de l'invention, le mélange réactionnel gazeux comprenant de l'acide (méth)acrylique obtenu par oxydation en phase gazeuse d'un précurseur de l'acide (méth)acrylique comprend du carbone d'origine renouvelable.

Selon un mode réalisation de l'invention, le précurseur de l'acide (méth)acrylique est dérivé du glycérol, de l'acide 3-hydroxypropionique ou de l'acide 2-hydroxypropanoïque (acide lactique).

Selon un mode réalisation préféré de l'invention, le mélange réactionnel gazeux comprend de l'acide acrylique dérivé de propylène obtenu selon un procédé d'oxydation en deux étapes.

Le procédé selon l'invention produit un flux d'acide (méth)acrylique sans produire de rejets aqueux, et ne nécessite pas l'utilisation d'un solvant azéotropique pour éliminer l'eau du procédé. Le procédé selon l'invention contribue également à limiter les pertes en acide (méth)acrylique en tête de la colonne de déshydratation.

La présente invention a également pour objet une installation pour récupérer de l'acide (méth)acrylique, adaptée pour mettre en oeuvre le procédé selon l'invention.

L'installation selon l'invention comprend au moins :
a) une colonne de déshydratation ;
b) une colonne de finition connectée fluidiquement en pied de ladite colonne de déshydratation ;
c) au moins un système de condensation à pompe à vide sèche connecté fluidiquement en tête de ladite colonne de finition.
d) de façon optionnelle un système de condensation à pompe à vide sèche E230/300 connecté fluidiquement latéralement à ladite colonne de finition.

Par « connexion fluidique » ou « connecté fluidiquement », on veut indiquer qu'il y a connexion par un système de conduites aptes à transporter un flux de matière. Ce système de connexion peut comprendre des vannes, des dérivations, des échangeurs de chaleur ou des compresseurs.

Selon certains modes de réalisation particuliers, l'invention présente également une ou, de préférence, plusieurs des caractéristiques avantageuses énumérées ci-dessous :
- le système de condensation à pompe à vide sèche comprend au moins un condenseur connecté fluidiquement à une pompe à vide sèche ;
- le système de condensation à pompe à vide sèche comprend plusieurs condenseurs ;
- le système de condensation à pompe à vide sèche peut comprendre de façon optionnelle, un séparateur de liquide, un ou plusieurs pare-flamme, un ou plusieurs filtres, des systèmes d'étanchéité et d'isolement, une pompe ou une combinaison de pompes sèches d'appoint, comme par exemple des pompes dites Roots ;
- La colonne de finition est connectée fluidiquement à un système de condensation à pompe à vide sèche, en tête de colonne, ou en tête de colonne et latéralement à la colonne.
- le système de condensation à pompe à vide sèche est connecté fluidiquement à la colonne de finition pour condenser un flux distillé en tête de la colonne de finition, ou pour condenser un flux soutiré latéralement de la colonne de finition, ou un mélange de ces deux flux.

Un autre objet de l'invention est un procédé de production d'acide (méth)acrylique comprenant au moins les étapes suivantes :
A) on soumet à une oxydation en phase gazeuse au moins un précurseur d'acide (méth)acrylique pour former un mélange réactionnel gazeux comprenant de l'acide (méth)acrylique ;
B) on refroidit le mélange réactionnel gazeux ;
C) on soumet le mélange réactionnel gazeux refroidi au procédé de récupération de l'acide (méth)acrylique tel que défini précédemment.

La présente invention permet de surmonter les inconvénients de l'état de la technique liés à la mise en oeuvre d'un procédé de récupération/purification sans solvant que nécessite l'obtention d'un acide (méth)acrylique purifié.

D'autres caractéristiques et avantages de l'invention ressortiront mieux à la lecture de la description détaillée qui suit, en référence aux figures 1 à 4 annexées qui représentent :
- Figure 1 : schéma d'un procédé de production d'acide acrylique illustrant le procédé de récupération/purification sans solvant selon l'invention.
- Figure 2 : installation adaptée à la mise en oeuvre du procédé de récupération illustrant un mode de réalisation de l'invention.
- Figure 3 : installation de l'art antérieur incluant un système de condensation à pompe à anneau liquide.
- Figure 4 : installation de l'art antérieur avec un système de condensation intégrant des éjecteurs de vapeur.

### DESCRIPTION DETAILLEE DE L'INVENTION

L'invention est basée sur l'intégration d'une pompe à vide sèche connectée fluidiquement à un condenseur formant un système de condensation à pompe à vide sèche dans un procédé pour produire de l'acide (méth)acrylique.

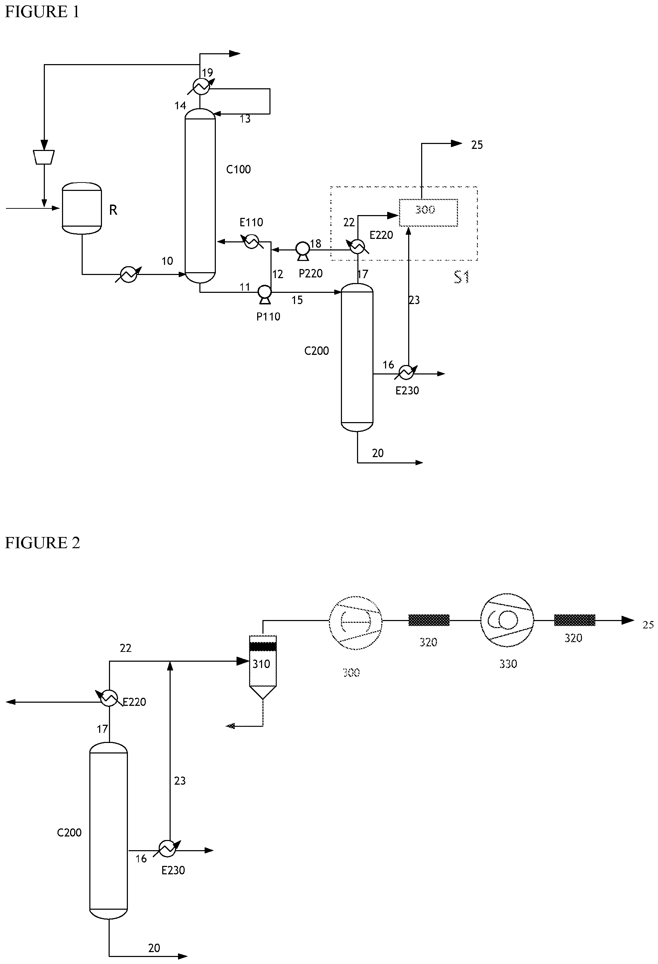

Sur la Figure 1, on voit un réacteur R produisant un mélange réactionnel gazeux 10 comprenant de l'acide (méth)acrylique obtenu par oxydation en phase gazeuse d'un précurseur de l'acide (méth)acrylique. Le mélange réactionnel gazeux comportant un rapport massique eau/acide (méth)acrylique généralement compris entre 0,3 et 2 peut être préalablement refroidi avant d'être soumis à une déshydratation selon l'étape i) du procédé selon l'invention dans une première colonne C100 dite colonne de déshydratation. La colonne de déshydratation conduit à un flux de tête 14 dont une partie est condensée et renvoyée à la colonne de déshydratation sous forme de reflux 13 pour absorber l'acide (méth)acrylique, l'autre partie 19 comprenant les composés légers incondensables étant généralement envoyée partiellement ou totalement à un dispositif d'épuration ou recyclée en partie vers d'autres étapes du procédé de production d'acide (méth)acrylique, de préférence dans une étape située en amont du réacteur R.

Selon un mode de réalisation, le réacteur R est un ensemble de 2 réacteurs en série ou comprend au moins 2 zones réactionnelles en série, le premier réacteur ou la première zone de réaction étant utilisée pour la synthèse du précurseur de l'acide (méth)acrylique.

Selon un mode de réalisation, la totalité du flux de tête 14 de la colonne de déshydratation est envoyée dans le condenseur de tête.

Le but de l'étape i) est d'éliminer dans un flux de tête l'essentiel de l'eau présente dans le mélange réactionnel, mais aussi les composés légers incondensables et les composés légers condensables. La colonne de déshydratation fonctionne, au moins partiellement, comme une colonne de distillation. Elle est alimentée dans sa partie inférieure par le mélange réactionnel 10. Elle génère un flux de tête 14 comprenant l'essentiel de l'eau et des composés légers, ce flux de tête étant appauvri en acide (méth)acrylique, et un flux de pied 11 comprenant l'essentiel de l'acide (méth)acrylique avec des sous-produits lourds.

Avantageusement, la colonne de déshydratation fonctionne à la pression atmosphérique ou légèrement supérieure, jusque 1,5 10⁵ Pa.

Avantageusement, la température dans la partie supérieure de la colonne de déshydratation est d'au moins 40°C, de préférence est comprise entre 40°C et 80°C. La température du flux de pied de la colonne de déshydratation ne dépasse pas de préférence 120°C.

Selon l'invention, l'essentiel de l'eau présente dans le mélange réactionnel gazeux comprenant l'acide (méth)acrylique est éliminé au cours de l'étape i) sans qu'il n'y ait de perte excessive d'acide acrylique dans le flux de tête 19.

Aucun solvant azéotropique n'est ajouté dans la colonne de déshydratation.

La teneur massique en eau dans le flux de pied de la colonne de déshydratation est généralement inférieure à 10%, de préférence inférieure à 7%.

Selon l'étape ii) du procédé selon l'invention, le flux de pied 11 de la colonne de déshydratation est envoyé au moins en partie (flux 15), en tête d'une seconde colonne de distillation, dite colonne de finition, ou colonne de purification, C200, dans laquelle sont séparés un flux de tête 17 et un flux de pied 20.

En alternative, le flux de pied de la colonne de déshydratation est envoyé au moins en partie entre la tête et le soutirage latéral de la colonne de purification.

Le flux de pied de la colonne de déshydratation peut passer en partie dans un bac intermédiaire avant de pénétrer dans la colonne de purification.

Selon un mode de réalisation, une partie 12 du flux liquide 11 de pied de la colonne de déshydratation est envoyée par une pompe P110 dans un échangeur de chaleur E110, qui peut être un réchauffeur ou un refroidisseur et réinjectée dans la colonne de déshydratation, de façon à constituer une boucle de pied. De préférence, la partie 12 du flux de pied est réinjectée entre l'alimentation du mélange gazeux réactionnel et la tête de colonne de déshydratation. Le reste (flux 15) du flux liquide 11 est envoyée par la même pompe P110 en alimentation de la colonne de finition (ou purification) C200.

La colonne de déshydratation et la colonne de finition peuvent être de configurations diverses, par exemple de type colonne à garnissage vrac ou structuré ou colonnes à plateaux.

La colonne de déshydratation comprend généralement de 5 à 50 plateaux théoriques, de préférence de 20 à 30 plateaux théoriques ; la colonne de finition comprend généralement de 5 à 30 plateaux théoriques, de préférence de 8 à 20 plateaux théoriques. Le choix du type d'internes dans les colonnes et le choix des équipements annexes tels que échangeurs de chaleur, condenseurs, pompes, entrée et sorties des fluides seront facilement déterminés selon les considérations connues de l'homme de l'art.

La colonne de finition (ou purification) est une colonne de distillation associée à un rebouilleur et un condenseur.

La température et la pression dans la colonne de purification ne sont pas critiques, et peuvent être déterminées conformément aux méthodes de distillation connues de l'état de l'art. Cependant, de préférence, la colonne de purification fonctionne à une pression inférieure à la pression atmosphérique, permettant de fonctionner à des températures relativement faibles, évitant ainsi la polymérisation des produits insaturés présents, et minimisant la formation de sous-produits lourds.

Avantageusement, la colonne de purification fonctionne sous une pression allant de 5 kPa à environ 60 kPa, la température du flux de tête étant avantageusement comprise entre 40°C et environ 90°C, et la température du flux de pied étant comprise ente 60°C et 120°C.

Selon le procédé de récupération de l'invention, le flux gazeux de tête 17 de la colonne de finition C200 est envoyé au moins en partie, de préférence en totalité, dans un système S1 de condensation à pompe à vide sèche, représenté sur la Figure 1 par l'ensemble constitué par le condenseur E220 et la pompe à vide sèche 300. Le liquide condensé 18 contenant principalement des composés légers, en particulier eau et acide acétique, ainsi que de l'acide (méth)acrylique, est avantageusement recyclé via une pompe P220, dans la colonne de déshydratation C100. L'évent 22 non condensé en sortie du condenseur E220 est introduit dans la pompe sèche 300, avant d'être éliminé sous la forme d'un effluent gazeux ultime 25.

L'utilisation d'un tel système S1 intégrant une pompe à vide sèche fournit une pression en-dessous de la pression atmosphérique dans la colonne de finition, permettant ainsi d'éliminer à température réduite les composés légers résiduels issus de l'étape antérieure de déshydratation du mélange réactionnel comprenant l'acide (méth)acrylique.

Aucun effluent aqueux n'est produit par le système de condensation à pompe à vide sèche.

Des exemples de pompes à vide sèches à piston sont décrits par exemple dans les documents US 2005/260085 ou US 5,921,755. Les pompes à vide sèches peuvent également être composées par exemple d'un corps cylindrique dans lequel tourne un rotor en position excentrique, doté d'entailles dans lesquelles sont insérées des palettes permettant d'aspirer un flux gazeux. Tout autre type de configuration peut être utilisé comme pompe à vide sèche. Le terme « sèche » indique qu'aucun flux liquide, tel qu'une huile lubrifiante ou de l'eau, n'est en contact avec le flux gazeux alimentant la pompe.

Comme exemples de pompe à vide sèche, on peut citer, sans que cette liste soit limitative, des pompes mécaniques sèches à vis, à soufflets d'étanchéité, à spirales (pompes scroll), à piston rotatif, à lobe rotatif, à membrane sans huile, par exemple les pompes à vide sèches à vis commercialisées par la société Edwards, ou les pompes à rotors, commercialisées par la société Sihi. Ces pompes créent un vide primaire qui peut si nécessaire être complété par d'autres pompes à vide sèches, comme par exemple des pompes sèches d'appoint de type « Roots ».

Avantageusement, le vide obtenu dans la colonne de finition peut être modulé en fonction de la vitesse de fonctionnement de la pompe à vide sèche.

Un flux d'acide (méth)acrylique 16 est récupéré par soutirage latéral à partir de la colonne de finition (étape iii), à un niveau latéral situé de préférence en-dessous de l'alimentation de ladite colonne. Le flux de produit 16 soutiré peut être un flux liquide ou un flux gazeux.

Le flux 16 soutiré latéralement correspond à un grade d'acide (méth)acrylique technique. Il est en général constitué d'acide (méth)acrylique de pureté supérieure à 98%, de préférence supérieure à 99%. De préférence, il contient moins de 1,5%, de préférence moins de 0,5%, plus particulièrement moins de 0,2% en poids d'acide acétique, et moins de 1%, de préférence moins de 0,5%, plus particulièrement, moins de 0,3% en poids d'eau.
Le flux 16 peut encore être soumis à une purification par distillation, éventuellement couplée avec un traitement de cristallisation.

Selon un mode de réalisation préféré de l'invention, au moins une partie du flux d'acide (méth)acrylique purifié 16 soutiré latéralement est soumis à un système de condensation à pompe à vide sèche qui peut comprendre la pompe à vide sèche 300 utilisée en tête de la colonne de finition.

Selon un mode de réalisation, le flux 16 est soumis à une condensation dans un condenseur E230 et le flux gazeux 23 est envoyé dans la pompe à vide 300.

Selon un mode de réalisation particulier de l'invention, comme représenté à la Figure 2, le flux gazeux 22, éventuellement mélangé avec le flux 23, alimentant la pompe à vide sèche, passe préalablement dans un dispositif 310 séparateur de liquide, permettant de séparer les traces de liquide résiduel, riche en composés légers, en particulier eau et acide acétique, ainsi que de l'acide (méth)acrylique résiduel. De manière préférentielle, ce flux liquide sera recyclé dans la colonne de déshydratation, par exemple en mélange avec le flux 18 alimentant la pompe P220.

Selon d'autres modes de réalisation, le gaz 25 en sortie de la pompe à vide sèche passe par différents éléments, tels que ceux décrits précédemment, en particulier au moins un pare-flamme 320, et une pompe sèche d'appoint 330, avant d'être envoyé finalement dans un incinérateur.

Le condensat 18, formé par le système de condensation à pompe à vide sèche, est avantageusement renvoyé en partie ou en totalité vers la colonne de déshydratation, entre le pied et la tête de la colonne et de préférence au-dessus de l'alimentation du mélange gazeux réactionnel. Selon un mode de réalisation il est mélangé au flux 12 de la boucle de pied de la colonne de déshydratation, comme représenté sur la Figure 1.

Optionnellement, le flux 18 peut transiter dans un bac de stockage intermédiaire avant recyclage dans la boucle de pied de colonne de déshydratation.

Un flux d'acide (méth)acrylique 20 comprenant l'essentiel des sous-produits lourds, notamment des produits d'addition de Michael ainsi que des inhibiteurs de polymérisation, est récupéré en pied de la colonne de finition (étape iii).

Le flux de pied 20 de la colonne de finition correspond à un grade d'acide (méth)acrylique brut qui peut être utilisé directement comme matière première dans une unité de production d'esters acryliques par estérification directe, ou éventuellement après une étape de décomposition thermique des dérivés d'addition de Michael libérant de l'acide (meth)acrylique. Alternativement, le flux de pied 20 peut être purifié dans une troisième colonne de distillation pour obtenir un acide (méth)acrylique de grade technique.

Avantageusement, le flux 16 de produit soutiré latéralement et le flux 20 de pied de la colonne de finition sont récupérés selon un rapport massique allant de 99 : 1 à 25 : 75, de préférence de 98 : 2 à 50 : 50.

Des inhibiteurs de polymérisation peuvent être introduits à différents endroits dans l'installation de mise en oeuvre du procédé de l'invention, notamment dans le flux de tête de la colonne de déshydratation au niveau du condenseur, ou dans le flux de tête de la colonne de purification au niveau du condenseur associé à ladite colonne, ou dans le flux de produit purifié soutiré latéralement de la colonne de purification, éventuellement après condensation dans le cas où le flux soutiré est sous forme gazeuse.

Les inhibiteurs de polymérisation sont choisis parmi les composés qui inhibent la réaction de polymérisation de l'acide (méth)acrylique et sont ajoutés en quantité suffisante connue de l'homme de l'art pour éviter ou réduire la polymérisation de l'acide (méth)acrylique. Comme exemples de composés utilisables, on peut citer la phénothiazine, l'hydroquinone, le 2,2,6,6-tétraméthyl-1-pipéridinyloxy (Tempo) ou l'un de ses dérivés tel que le 4-hydroxy Tempo, les sels de cuivre solubles, les sels de manganèse solubles, seuls ou en mélange, éventuellement en solution dans l'eau, dans l'acide (méth)acrylique ou dans un mélange d'eau et d'acide (méth)acrylique.

Selon un mode de réalisation de l'invention, la nature de l'inhibiteur varie selon l'endroit où il est injecté.

Selon un mode de réalisation de l'invention, de l'air ou un gaz comprenant de l'oxygène est introduit, par exemple dans les pieds des colonnes de déshydratation et de purification, dans les rebouilleurs des colonnes, dans la boucle de recirculation en pied de colonne de déshydratation ou au niveau du soutirage latéral de la colonne de purification ou dans les condenseurs.

Le procédé de l'invention fournit directement en soutirage latéral de la colonne de finition, une qualité d'acide (méth)acrylique qui correspond à un grade d'acide (méth)acrylique technique, qui peut être envoyé ensuite sur une unité de purification, par exemple par cristallisation, pour obtenir une qualité d'acide (méth)acrylique dite « glaciale ». Il fournit également en pied de la colonne de finition, une qualité d'acide (méth)acrylique brut qui peut ensuite être purifié ou traité thermiquement pour obtenir un grade d'acide (méth)acrylique technique.

L'installation selon l'invention adaptée pour mettre en oeuvre le procédé de récupération/purification de l'acide (méth)acrylique tel que décrit, comprend au moins :
a) une colonne de déshydratation C100 ;
b) une colonne de finition C200 connectée fluidiquement en pied de ladite colonne de déshydratation ;
c) au moins un système S1 de condensation à pompe à vide sèche E220/300, connecté fluidiquement en tête de ladite colonne de finition.
d) de façon optionnelle un système de condensation à pompe à vide sèche E230/300 connecté fluidiquement latéralement à ladite colonne de finition.

Un autre objet de l'invention porte sur un procédé de production d'acide (méth)acrylique comprenant au moins les étapes suivantes :
A) on soumet à une oxydation en phase gazeuse au moins un précurseur d'acide (méth)acrylique pour former un mélange réactionnel gazeux comprenant de l'acide (méth)acrylique ;
B) on refroidit le mélange réactionnel gazeux ;
C) on soumet le mélange réactionnel gazeux refroidi au procédé de récupération de l'acide (méth)acrylique tel que défini précédemment.

Le précurseur de l'acide (méth)acrylique peut être l'acroléine ou la méthacroléine, et peut être dérivé de matière première renouvelable produisant ainsi de l'acide (méth)acrylique biosourcé.

De préférence, l'acide (méth)acrylique est l'acide acrylique et le précurseur de l'acide acrylique est l'acroléine obtenu par oxydation catalytique de propylène.

La réaction d'oxydation de l'étape A), effectuée selon les connaissances de l'art, fournit généralement un mélange réactionnel gazeux, surchauffé à une température supérieure à 280°C.

Ce mélange est avantageusement refroidi selon une étape B), notamment jusqu'à une température inférieure à 250°C, de préférence inférieure à 190°C, pour être soumis selon l'étape C) au procédé de récupération de l'acide (méth)acrylique sans utiliser de solvant azéotropique incluant un système de condensation à pompe à vide sèche. Il peut être refroidi directement dans la colonne de déshydratation, ou peut être refroidi à l'aide d'un échangeur de chaleur situé en amont de la colonne de déshydratation.

Même si l'utilisation du système de condensation à pompe à vide sèche est décrit dans la présente invention dans un procédé de production d'acide (méth)acrylique incluant un processus de purification sans solvant avec deux colonnes de distillation, le système de condensation à pompe à vide sèche peut être utilisé également dans d'autres procédés qui produisent un mélange réactionnel gazeux, afin de réduire la quantité d'eau et de vapeur utilisée, et réduire ainsi la quantité d'effluents aqueux rejetés.

L'invention sera maintenant illustrée par les exemples suivants, qui n'ont pas pour but de limiter la portée de l'invention, définie par les revendications annexées.

### PARTIE EXPERIMENTALE

### Exemple 1 (selon l'invention)

Des simulations à l'aide du logiciel ASPEN ont été utilisées pour illustrer le procédé selon l'invention.

En référence à la Figure 1, dans un procédé de production en continu d'acide acrylique à partir de propylène, un mélange réactionnel 10 a été soumis au procédé de récupération/purification selon l'invention.

Dans ce procédé, 11 000 kg/h d'acide acrylique technique sont produits (flux 16), ayant une pureté de 99,8%. Les principales impuretés sont l'acide acétique (0,05%), l'acide propionique, (0,021%), le furfural (0,014%), le benzaldéhyde (0,008%), l'anhydride maléique (0,037%).

Le flux gazeux 22 (67,6 kg/h) provenant du condenseur E220 en tête de la colonne de finition C200 a été soumis à une pompe à vide sèche 300. Cette pompe assure une pression de 12 kPa en tête de la colonne C200. Un flux 23 (7,3 kg/h) provenant du condenseur E230 placé à la sortie latérale du flux d'acide acrylique technique a été envoyé en même temps à la pompe à vide sèche 300.

Les principaux constituants des flux 22 et 23 entrants, ainsi que la composition du flux gazeux 25 sortant, exprimée en débit massique horaire (kg/h), sont rassemblés dans le tableau 1.

Aucun effluent aqueux n'est produit selon ce système.

A la sortie de la pompe à vide sèche, seul un effluent gazeux ultime 25 (74,9 kg/h) est émis, qui est facile à éliminer par les méthodes connues de l'homme de l'art, par exemple par oxydation thermique ou catalytique.

La perte d'acide acrylique dans le flux 19 refroidi à 57°C est de 0,69%, soit un rendement de récupération d'acide acrylique de 99,31%.

**Tableau 1**

| Débit massique kg/h | **Flux 22** | **Flux 23** | **Flux 25 effluent gazeux** |
|---|---|---|---|
| N₂ | 4,823E+00 | 0,000E+00 | 4,823E+00 |
| O₂ | 3,032E+01 | 6,134E+00 | 3,646E+01 |
| CO | 2,441E-02 | 0,000E+00 | 2,441E-02 |
| CO₂ | 1,431E-01 | 0,000E+00 | 1,431E-01 |
| propylène | 6,118E-01 | 0,000E+00 | 6,118E-01 |
| propane | 1,990E-01 | 0,000E+00 | 1,990E-01 |
| formaldéhyde | 7,109E-01 | 0,000E+00 | 7,109E-01 |
| acétaldéhyde | 6,608E-03 | 0,000E+00 | 6,608E-03 |
| acroléine | 3,537E-01 | 0,000E+00 | 3,537E-01 |
| H₂O | 8,108E+00 | 2,316E-09 | 8,108E+00 |
| Acide acétique | 4,870E+00 | 1,441E-03 | 4,871E+00 |
| Acide acrylique | 1,739E+01 | 1,181E+00 | 1,857E+01 |
| Acide propionique | 1,941E-03 | 2,357E-04 | 2,177E-03 |
| furfural | 6,446E-04 | 9,564E-05 | 7,402E-04 |
| benzaldéhyde | 4,463E-04 | 3,563E-05 | 4,820E-04 |
| Anhydride maléïque | 5,412E-04 | 7,153E-05 | 6,127E-04 |
| **TOTAL** | **67,571** | **7,316** | **74,887** |

### Exemples 2 et 3 (comparatifs)

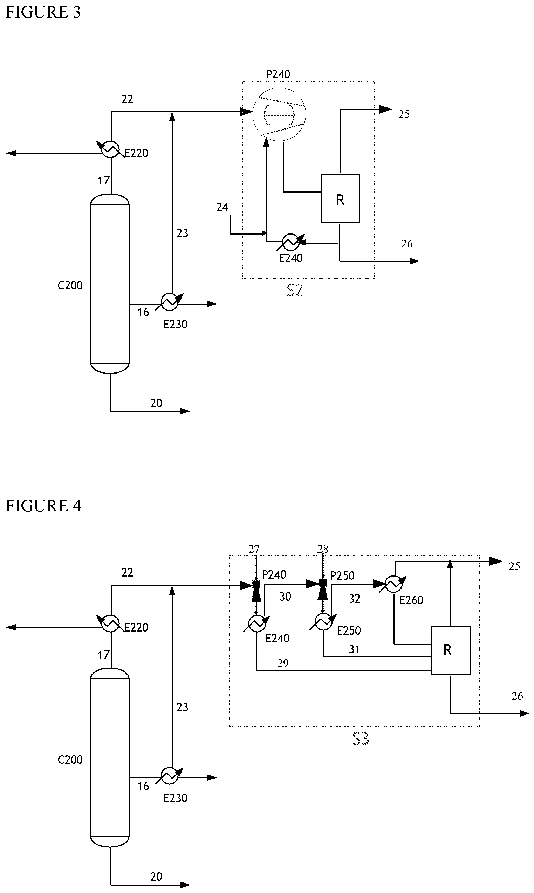

A titre de comparaison, les mêmes flux 22 et 23 ont été soumis respectivement à une pompe à anneau liquide (exemple 2, Figure 3) en remplacement de la pompe à vide sèche, et à un système intégrant des éjecteurs de vapeur (exemple 3, Figure 4).

En particulier, l'exemple 3 met en jeu une technologie de générateur de vide par éjecteur de vapeur décrite dans les documents US 6,677,482 ou US 7,288,169.

Sur la Figure 3, est représenté un système S2 intégrant une pompe à anneau liquide P240, alimentée par un flux d'eau 24. Le débit du flux 24 introduit dans la pompe pour assurer une pression de 12 kPa est 1000 kg/h. Ce système S2 comprend une pompe P240, un échangeur E240 et un récipient de collecte des condensats R.

La pompe P240 est alimentée d'une part par les flux gazeux 22 et 23 provenant de la colonne C200 et d'autre part par le flux aqueux 27. Le rôle principal de ce flux aqueux est de constituer un joint liquide nécessaire à la génération de la dépression dans la pompe et d'assurer le renouvellement du liquide en le purgeant des impuretés condensées. La chaleur dégagée par la pompe est éliminée grâce au refroidissement du flux condensé à travers l'échangeur.

Le flux sortant de la pompe est en partie liquide et en partie gazeux. Les 2 phases sont séparées dans le récipient R et une partie de la phase liquide (essentiellement aqueuse) est recirculée vers la pompe P240 après refroidissement dans l'échangeur E240.

Ce système S2 produit donc en sortie un flux gazeux 25 (43,2 kg/h), mais aussi un effluent liquide 26 en quantité importante (1031,7 kg/h). Ce flux 26, essentiellement aqueux, contient des composés organiques en solution, à des concentrations importantes (principalement 1,8% d'acide acrylique, 0,5% d'acide acétique), qui le rendent impropre au rejet sans traitement complémentaire de purification.

Sur la Figure 4, est représenté un système S3 intégrant deux éjecteurs en série P240 et P250, alimentés en respectivement par 400 kg/h de vapeur d'eau (flux 27) et 600kg/h de vapeur d'eau (flux 28), de façon à assurer une pression de 12 kPa en tête de la colonne C200.

Ce système S3 comprend les 2 pompes (éjecteurs) montées en série qui sont alimentées par de la vapeur à pression de 1500 kPa et 3 condenseurs.

Le premier éjecteur P240 est alimenté d'une part par les flux gazeux 22 et 23 provenant de la colonne C200 et d'autre part par le flux de vapeur d'eau sous pression. Le flux gazeux sortant à température de 144°C est refroidi à température de 42°C dans un premier condenseur E240. Le condensat liquide 29 est envoyé à un récipient de collecte des condensats R et les évents gazeux non condensés 30 sont envoyés à l'alimentation du 2^{ème} éjecteur P250. En sortie de cet éjecteur, le flux gazeux à 162°C est refroidi à température de 42°C dans le condenseur E250. Le flux liquide condensé 31 est envoyé dans le récepteur R. Les évents 32 non condensés dans ce 2^{ème} condenseur sont refroidis à 15°C dans le 3^{ème} condenseur E260, produisant un 3^{ème} condensat liquide recueilli dans le réservoir R. Le flux gazeux 25 non condensé dans ce 3^{ème} condenseur est éliminé.

Ce système S3 produit ainsi en sortie un flux gazeux 25 (42,8 kg/h), mais aussi un effluent aqueux 26 en quantité importante (1032,1 kg/h). Ce flux aqueux 26 contient des composés organiques en solution, à des concentrations importantes (principalement 1,8% d'acide acrylique, 0,5% d'acide acétique), qui le rendent impropre au rejet sans traitement complémentaire de purification.

Les principaux constituants du flux gazeux 25 et du flux aqueux 26 en sortie des systèmes S2 et S3 sont indiqués dans le tableau 2.

**Tableau 2**

| | **Exemple 1** | **Exemple 2 (comp)** | | **Exemple 3 (comp)** | |
|---|---|---|---|---|---|
| Débit massique kg/h | **Flux gazeux 25** | **Flux gazeux 25** | **Flux aqueux 26** | **Flux gazeux 25** | **Flux aqueux 26** |
| N₂ | 4,823E+00 | 4,820E+00 | 2,845E-03 | 4,821E+00 | 1,453E-03 |
| O₂ | 3,646E+01 | 3,642E+01 | 3,737E-02 | 3,644E+01 | 1,931E-02 |
| CO | 2,441E-02 | 2,439E-02 | 1,782E-05 | 2,439E-02 | 1,207E-05 |
| CO₂ | 1,431E-01 | 1,376E-01 | 5,575E-03 | 1,410E-01 | 2,102E-03 |
| propylène | 6,118E-01 | 6,076E-01 | 4,248E-03 | 6,107E-01 | 1,148E-03 |
| propane | 1,990E-01 | 1,987E-01 | 2,888E-04 | 1,989E-01 | 1,124E-04 |
| formaldéhyde | 7,109E-01 | 9,219E-04 | 7,099E-01 | 5,115E-05 | 7,108E-01 |
| acétaldéhyde | 6,608E-03 | 8,909E-04 | 5,717E-03 | 1,830E-03 | 4,778E-03 |
| acroléine | 3,537E-01 | 9,535E-02 | 2,584E-01 | 1,591E-01 | 1,946E-01 |
| H₂O | 8,108E+00 | 8,758E-01 | 1,007E+03 | 4,162E-01 | 1,008E+03 |
| Acide acétique | 4,871E+00 | 2,005E-03 | 4,869E+00 | 1,634E-05 | 4,871E+00 |
| Acide acrylique | 1,857E+01 | 7,855E-03 | 1,857E+01 | 6,686E-05 | 1,857E+01 |
| Acide propionique | 2,177E-03 | 1,239E-06 | 2,175E-03 | 1,878E-08 | 2,177E-03 |
| furfural | 7,402E-04 | 4,273E-06 | 7,360E-04 | 2,027E-06 | 7,382E-04 |
| Benzaldéhyde | 4,820E-04 | 3,393E-05 | 4,480E-04 | 5,944E-05 | 4,225E-04 |
| Anhydride maléïque | 6,127E-04 | 5,197E-08 | 6,127E-04 | 2,376E-11 | 6,127E-04 |
| **TOTAL** | **74,887** | **43,191** | **1031,697** | **42,811** | **1032,075** |

Les systèmes classiques sous vide mettant en jeu une pompe à anneau liquide, ou des éjecteurs à jet de vapeur génèrent une quantité importante d'effluent aqueux. Cet effluent contient des impuretés organiques et doit donc être traité.

### Exemple 4

Pour éviter le traitement coûteux du flux aqueux issu de la pompe à anneau liquide (exemple 2) ou du système à éjecteurs (exemple 3), et récupérer une partie de l'acide acrylique contenu dans ces flux, il peut être envisagé de recycler ces flux dans le procédé de purification sans solvant.

Une simulation du procédé de purification sans solvant a été réalisée à l'aide du logiciel ASPEN, intégrant un recyclage du flux aqueux 26 condensé dans l'exemple 3 (éjecteurs en série), en mélange dans le flux 18 renvoyé vers la colonne de déshydratation C100 (voir Figure 1).

Dans ces conditions, contrairement à l'objectif recherché, on observe que le recyclage de ce flux aqueux (1032 kg/h) occasionne une perte significative d'acide acrylique en tête de colonne C100 de condensation.

La perte d'acide acrylique dans le flux 19 refroidi à 61°C est de 1,74%, soit un rendement de récupération d'acide acrylique de 98,26%.

## Revendications

1. Procédé de récupération d'acide (méth)acrylique sans utiliser de solvant azéotropique, à partir d'un mélange réactionnel gazeux comprenant de l'acide (méth)acrylique obtenu par oxydation en phase gazeuse d'un précurseur de l'acide (méth)acrylique, comprenant au moins les étapes suivantes :
i) on soumet le mélange réactionnel gazeux à une déshydratation sans utiliser de solvant azéotropique dans une première colonne dite colonne de déshydratation, conduisant à un flux de tête dont une partie au moins est condensée et renvoyée à la colonne de déshydratation sous forme de reflux, et à un flux de pied ;
ii) on soumet au moins en partie le flux de pied de colonne de déshydratation à une distillation à une pression inférieure à la pression atmosphérique dans une seconde colonne dite colonne de finition, conduisant à un flux de tête, et à un flux de pied contenant des composés lourds ;
iii) on récupère un flux d'acide (méth)acrylique par soutirage latéral de la colonne de finition, et/ou en pied de la colonne de finition ;
ledit procédé étant **caractérisé en ce que** le flux de tête de la colonne de finition est au moins en partie soumis à un système de condensation à pompe à vide sèche, formant un condensat qui est renvoyé dans la colonne de déshydratation, et un effluent gazeux ultime.

2. Procédé selon la revendication 1 **caractérisé en ce que** le système de condensation à pompe à vide sèche comprend au moins un condenseur et une pompe à vide sèche.

3. Procédé selon la revendication 1 ou 2 **caractérisé en ce que** le système de condensation à pompe à vide sèche comprend en outre un séparateur de liquide, ou un ou plusieurs pare-flamme, ou un ou plusieurs filtres, ou des systèmes d'étanchéité et d'isolement, ou une pompe ou une combinaison de pompes sèches d'appoint.

4. Procédé selon l'une quelconque des revendications précédentes **caractérisé en ce qu'**au moins une partie du flux d'acide (méth)acrylique soutiré latéralement est soumis à un système de condensation à pompe à vide sèche, identique ou différent de celui utilisé pour le flux de tête de la colonne de finition.

5. Procédé selon l'une quelconque des revendications précédentes **caractérisé en ce que** le précurseur de l'acide (méth)acrylique est l'acroléine, obtenue par oxydation de propylène ou par oxydéshydrogénation de propane.

6. Procédé selon l'une quelconque des revendications 1 à 4 **caractérisé en ce que** le précurseur de l'acide (méth)acrylique est la méthacroléine obtenue par oxydation d'isobutylène et/ou de tert-butanol ou à partir d'oxydéshydrogénation de butane et/ou isobutane.

7. Procédé selon l'une quelconque des revendications 1 à 4 **caractérisé en ce que** le précurseur de l'acide (méth)acrylique comprend du carbone d'origine renouvelable.

8. Procédé selon la revendication 7 **caractérisé en ce que** le précurseur de l'acide (méth)acrylique est dérivé du glycérol, de l'acide 3-hydroxypropionique ou de l'acide 2-hydroxypropanoïque.

9. Procédé selon l'une quelconque des revendications précédentes **caractérisé en ce qu'**il comprend en outre au moins une étape de purification du flux d'acide (méth)acrylique récupéré à l'étape iii).

10. Procédé de production d'acide (méth)acrylique comprenant au moins les étapes suivantes :
A) on soumet à une oxydation en phase gazeuse au moins un précurseur d'acide (méth)acrylique pour former un mélange réactionnel gazeux comprenant de l'acide (méth)acrylique ;
B) on refroidit le mélange réactionnel gazeux ;
C) on soumet le mélange réactionnel gazeux refroidi au procédé de récupération de l'acide (méth)acrylique tel que défini à l'une quelconque des revendications 1 à 9.

11. Procédé selon la revendication 10 **caractérisé en ce que** l'acide (méth)acrylique est l'acide acrylique et le précurseur de l'acide acrylique est l'acroléine obtenu par oxydation catalytique de propylène.

12. Installation pour récupérer de l'acide (méth)acrylique sans utiliser de solvant azéotropique comprenant au moins :
a) une colonne de déshydratation C100 ;
b) une colonne de finition C200 connectée fluidiquement en pied de ladite colonne de déshydratation ;
c) au moins un système de condensation à pompe à vide sèche E220/300, connecté fluidiquement en tête de ladite colonne de finition.
d) de façon optionnelle un système de condensation à pompe à vide sèche E230/300 connecté fluidiquement latéralement à ladite colonne de finition.

## Patentansprüche

1. Verfahren zur Gewinnung von (Meth)acrylsäure, ohne Verwendung eines azeotropen Lösungsmittels, aus einem gasförmigen Reaktionsgemisch, das (Meth)acrylsäure umfasst, die durch Oxidation in der Gasphase eines Vorläufers von (Meth)acrylsäure erhalten wird, umfassend mindestens die folgenden Schritte:
i) das gasförmige Reaktionsgemisch wird einer Dehydratisierung, ohne Verwendung eines azeotropen Lösungsmittels, in einer ersten Säule, die als Dehydratisierungssäule bezeichnet wird, unterzogen, wobei ein Kopfstrom, von dem mindestens ein Teil kondensiert und zu der Dehydratisierungssäule in der Form eines Rückflusses zurückgeschickt wird, und ein Fußstrom erhalten werden;
ii) der Fußstrom der Dehydratisierungssäule wird mindestens teilweise einer Destillation bei einem niedrigeren Druck als dem Atmosphärendruck in einer zweiten Säule, die als Endsäule bezeichnet wird, unterzogen, wobei ein Kopfstrom und ein Fußstrom erhalten werden, enthaltend schwere Verbindungen;
iii) ein Strom von (Meth)acrylsäure wird durch seitliche Entnahme aus der Endsäule und/oder am Fuß der Endsäule gewonnen;
wobei das Verfahren **dadurch gekennzeichnet ist, dass** der Kopfstrom der Endsäule mindestens teilweise einem Kondensationssystem mit einer Trockenvakuumpumpe unterworfen wird, wobei ein Kondensat, das in die Dehydratisierungssäule zurückgeschickt wird, und ein letztlicher Gasabfluss gebildet werden.

2. Verfahren nach Anspruch 1,
**dadurch gekennzeichnet, dass** das Kondensationssystem mit einer Trockenvakuumpumpe mindestens einen Kondensator und eine Trockenvakuumpumpe umfasst.

3. Verfahren nach Anspruch 1 oder 2,
**dadurch gekennzeichnet, dass** das Kondensationssystem mit einer Trockenvakuumpumpe außerdem einen Flüssigkeitsseparator oder ein oder mehrere Flammschutzmittel oder einen oder mehrere Filter oder Dichtungs- und Isoliersysteme oder eine Pumpe oder eine Kombination von Zusatztrockenpumpen umfasst.

4. Verfahren nach einem der vorhergehenden Ansprüche,
**dadurch gekennzeichnet, dass** mindestens ein Teil des Stroms von (Meth)acrylsäure, der seitlich entnommen wird, einem Kondensationssystem mit einer Trockenvakuumpumpe unterworfen wird, welches mit jenem, das für den Kopfstrom der Endsäule verwendet wird, identisch oder davon verschieden ist.

5. Verfahren nach einem der vorhergehenden Ansprüche,
**dadurch gekennzeichnet, dass** der Vorläufer von (Meth)acrylsäure Acrolein ist, das durch Oxidation von Propylen oder durch Oxydehydrierung von Propan erhalten wird.

6. Verfahren nach einem der Ansprüche 1 bis 4,
**dadurch gekennzeichnet, dass** der Vorläufer von (Meth)acrylsäure Methacrolein ist, das durch Oxidation von Isobutylen und/oder von tert.Butanol oder aus der Oxydehydrierung von Butan und/oder Isobutan erhalten wird.

7. Verfahren nach einem der Ansprüche 1 bis 4,
**dadurch gekennzeichnet, dass** der Vorläufer von (Meth)acrylsäure Kohlenstoff erneuerbaren Ursprungs umfasst.

8. Verfahren nach Anspruch 7,
**dadurch gekennzeichnet, dass** der Vorläufer von (Meth)acrylsäure von Glycerin, 3-Hydroxypropionsäure oder 2-Hydroxypropansäure stammt.

9. Verfahren nach einem der vorhergehenden Ansprüche,
**dadurch gekennzeichnet, dass** dieses außerdem mindestens einen Schritt der Reinigung des Stroms von (Meth)acrylsäure umfasst, der in Schritt iii) gewonnen wird.

10. Verfahren zur Herstellung von (Meth)acrylsäure, umfassend mindestens die folgenden Schritte:
A) mindestens ein Vorläufer von (Meth)acrylsäure wird einer Oxidation in der Gasphase unterzogen, um ein gasförmiges Reaktionsgemisch zu bilden, das (Meth)acrylsäure umfasst;
B) das gasförmige Reaktionsgemisch wird abgekühlt;
C) das abgekühlte gasförmige Reaktionsgemisch wird dem Verfahren zur Gewinnung von (Meth)acrylsäure, wie in einem der Ansprüche 1 bis 9 definiert, unterworfen.

11. Verfahren nach Anspruch 10,
**dadurch gekennzeichnet, dass** die (Meth)acrylsäure Acrylsäure ist, und der Vorläufer von Acrylsäure Acrolein ist, das durch katalytische Oxidation von Propylen erhalten wird.

12. Anlage zur Gewinnung von (Meth)acrylsäure, ohne Verwendung eines azeotropen Lösungsmittels, umfassend mindestens:
a) eine Dehydratisierungssäule C100;
b) eine Endsäule C200, die fluidisch am Fuß der Dehydratisierunssäule angeschlossen ist;
c) mindestens ein Kondensationssystem mit einer Trockenvakuumpumpe E220/300, die fluidisch am Kopf der Endsäule angeschlossen ist;
d) optional ein Kondensationssystem mit einer Trockenvakuumpumpe E230/300, die fluidisch seitlich von der Endsäule angeschlossen ist.

## Claims

1. Process for recovering (meth)acrylic acid without using azeotropic solvent, starting from a gaseous reaction mixture comprising (meth)acrylic acid obtained by gas-phase oxidation of a precursor of the (meth)acrylic acid, comprising at least the following steps:
i) the gaseous reaction mixture is subjected to dehydration without using azeotropic solvent in a first column, referred to as dehydration column, resulting in an overhead stream, at least a portion of which is condensed and sent back to the dehydration column in the form of reflux, and in a bottom stream;
ii) the dehydration column bottom stream is subjected, at least in part, to a distillation at a pressure below atmospheric pressure in a second column, referred to as finishing column, resulting in an overhead stream and in a bottom stream containing heavy compounds;
iii) a (meth)acrylic acid stream is recovered by drawing off as a sidestream from the finishing column, and/or as bottoms from the finishing column;
said process being **characterized in that** the overhead stream from the finishing column is subjected, at least in part, to a dry vacuum pump condensation system, forming a condensate that is sent back to the dehydration column, and a final gaseous effluent.

2. Process according to Claim 1, **characterized in that** the dry vacuum pump condensation system comprises at least one condenser and one dry vacuum pump.

3. Process according to Claim 1 or 2, **characterized in that** the dry vacuum pump condensation system additionally comprises a liquid separator, or one or more flame traps, or one or more filters, or sealing and insulating systems, or a dry booster pump or a combination of dry booster pumps.

4. Process according to any one of the preceding claims, **characterized in that** at least one portion of the (meth)acrylic acid stream drawn off as a sidestream is subjected to a dry vacuum pump condensation system, identical to or different from that used for the overhead stream from the finishing column.

5. Process according to any one of the preceding claims, **characterized in that** the precursor of the (meth)acrylic acid is acrolein, obtained by oxidation of propylene or by oxydehydrogenation of propane.

6. Process according to any one of Claims 1 to 4, **characterized in that** the precursor of the (meth)acrylic acid is methacrolein, obtained by oxidation of isobutylene and/or of tert-butanol or from oxydehydrogenation of butane and/or isobutane.

7. Process according to any one of Claims 1 to 4, **characterized in that** the precursor of the (meth)acrylic acid comprises carbon of renewable origin.

8. Process according to Claim 7, **characterized in that** that the precursor of the (meth)acrylic acid is derived from glycerol, 3-hydroxypropionic acid or 2-hydroxypropanoic acid.

9. Process according to any one of the preceding claims, **characterized in that** it additionally comprises at least one step of purifying the (meth)acrylic acid stream recovered in step iii).

10. Process for producing (meth)acrylic acid comprising at least the following steps:
A) at least one (meth)acrylic acid precursor is subjected to gas-phase oxidation in order to form a gaseous reaction mixture comprising (meth)acrylic acid;
B) the gaseous reaction mixture is cooled;
C) the cooled gaseous reaction mixture is subjected to the process for recovering (meth)acrylic acid as defined in any one of Claims 1 to 9.

11. Process according to Claim 10, **characterized in that** the (meth)acrylic acid is acrylic acid and the acrylic acid precursor is acrolein obtained by catalytic oxidation of propylene.

12. Plant for recovering (meth)acrylic acid without using azeotropic solvent, comprising at least:
a) one dehydration column C100;
b) one finishing column C200 fluidically connected to the bottom of said dehydration column;
c) at least one E220/300 dry vacuum pump condensation system, fluidically connected to the top of said finishing column;
d) optionally one E230/300 dry vacuum pump condensation system fluidically connected laterally to said finishing column.
